(19) **Europäisches Patentamt / European Patent Office / Office européen des brevets**

(11) **EP 4 667 806 A1**

(12) **EUROPEAN PATENT APPLICATION**
published in accordance with Art. 153(4) EPC

(43) Date of publication:
24.12.2025  Bulletin 2025/52

(21) Application number: 24756153.3

(22) Date of filing: 06.02.2024

(51) International Patent Classification (IPC):
*F16M 11/00* (2006.01)

(52) Cooperative Patent Classification (CPC):
F16M 11/00; F16M 11/04; F16M 11/10; F16M 11/16;
F16M 11/18

(86) International application number:
PCT/CN2024/076268

(87) International publication number:
WO 2024/169780 (22.08.2024 Gazette 2024/34)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority:  14.02.2023  CN 202310110698

(71) Applicant: Healinno (Beijing) Medical Technology
Co., Ltd.
Beijing 100176 (CN)

(72) Inventors:
• ZHAO, Jing
  Beijing 100176 (CN)
• LI, Hao
  Beijing 100176 (CN)
• SHEN, Lujia
  Beijing 100176 (CN)
• CHEN, Wenbo
  Beijing 100176 (CN)

(74) Representative: Metida
Gyneju str. 16
01109 Vilnius (LT)

(54) **ADJUSTMENT AND POSITIONING DEVICE FOR INSTRUMENT**

(57)     An adjustment and positioning device (100) for an instrument, comprising a support (1) and a locking mechanism (3). The support comprises a rack (13), and each tooth in the rack is provided with a tip (130). The locking mechanism is provided with a sliding member (30) and a plurality of locking insertion parts (31, 32), and the locking insertion parts abut against the sliding member by means of first elastic elements (35, 36). A surface tangent to tips of all teeth in the extending direction of the rack is regarded as a reference surface. It is assumed that the corresponding spacing between the centers of every two adjacent tips on the reference surface in the extending direction of the rack, i.e., the pitch, is $t_1$, and the corresponding spacing between the front ends of two adjacent locking insertion parts, which are randomly selected from the plurality of locking insertion parts, close to the rack on the reference surface in the extending direction of the rack, i.e., the insertion part spacing, is t2. t2 is a non-integer multiple of t1. The adjustment and positioning device for an instrument solves the problem of being unable to achieve stepless adjustment of the position of existing instruments, and improves the flexibility of position adjustment and positioning of the instruments.

FIG. 1

## Description

### CROSS-REFERENCE TO RELATED APPLICATIONS

The present application is based on, and claims the priority to Chinese Patent Application CN202310110698.1 (filed on February 14, 2023). The present application includes the entire contents of the application with reference to the application.

### TECHINCAL FIELD

The present application relates to an adjustment and positioning device for instrument, which is capable of limiting positional adjustment of an instrument to a specific degree of freedom while improving the flexibility of positional adjustment.

### BACKGROUND

For instruments requiring manual operation, such as medical instruments, the position and angle usually need to be manually adjusted during use. However, the precision of manual operation is difficult to control, and the hands of an operator may involuntarily tremble. Additionally, the subject acted upon by the instrument, such as human tissue also exerts a certain amount of force upon the instrument, inevitably causing unintended positional shifts. This results in reduced positioning accuracy, increased operational difficulty, and raised risks of errors.

To address this problem, in the prior art, the instrument is usually fixedly mounted on the adjusting and positioning device, thereby limiting the degree of freedom in positional adjustment. Therefore, even when there are a deviation in manual operation, hand tremors, or external forces acting on the instrument, the instrument can only follow the adjusting and positioning device to conduct a preset movement, thereby eliminating unnecessary disturbances in other degrees of freedom. Ideally, the movement of the instrument can be limited to a specific degree of freedom, thereby improving the accuracy of instrument adjusting and positioning.

The adjusting and positioning device usually includes a bracket with a rack, and the instrument is detachably fixed onto the bracket. Positional adjustment on the instrument is achieved through movement of the rack, and the locking mechanism engages with the rack to lock or unlock the instrument. However, for adjacent teeth of an arc-shaped rack, there is typically a specific included angle between them, while for adjacent teeth of a straight rack, there is generally a specific spacing. In the prior art, a gear, a rack, a worm, and other components are typically used as the locking mechanism to engage with the aforementioned rack. The problem with this design is that: typically, there is also a specific angle or spacing between teeth or plugs in the locking mechanism that engage with the rack, forcing the rack to adjust the position only in fixed steps. For example, when the rack is in an arc shape, the locking mechanism can be meshed with the rack only by rotating by integer multiples of the angle between two adjacent teeth, otherwise, the rack cannot be locked in the direction of rotation. As a result, the instrument cannot be adjusted in fine steps or adjusted steplessly, which increases the operational complexity and reduces the flexibility of positional adjustment.

Therefore, it has become a technical challenge in the prior art to enhance positional flexibility and further achieve stepless adjustment while limiting the positional adjustment on the instrument to a minimal degree of freedom.

### SUMMARY OF THE INVENTION

An objective of the present application is to provide an adjustment and positioning device for instrument which is capable of limiting positional adjustment of an instrument to a specific degree of freedom while improving the flexibility of positional adjustment, so as to achieve stepless adjustment to a certain extent. To achieve the forgoing objective, a solution is adopted in the present application: an adjustment and positioning device for instrument is provided, including a bracket fixedly connected with an instrument and a locking mechanism in locking engagement with the bracket, where the bracket of the adjustment and positioning device for instrument includes a rack disposed on a side face of the bracket; the locking mechanism includes a sliding member configured to move toward or away from the rack in a normal direction of the side face of the bracket provided with the rack; and a plurality of locking plugs connected to the sliding member and extending in the normal direction; by taking an arrangement direction of a plurality of teeth in the rack as an extension direction of the rack, and an end of each of the teeth close to the sliding member as a tooth tip, in the extension direction of the rack, each tooth further includes a first tooth surface and a second tooth surface extending from the tooth tip away from the sliding member, and tilting toward opposite sides of the normal direction; each of the plurality of locking plugs is connected to the sliding member via a first elastic element at a position further back relative to a tip end of the locking plug close to the rack in the extension direction of the locking plug, and the first elastic element allows a specified amount of deformation in the extension direction of the locking plug; under an elastic force of the first elastic element, the tip end of the locking plug close to the rack is capable of moving toward the rack and insert itself into a gap, i.e., tooth slot between two adjacent teeth of the rack; a surface tangent to tooth tips of all teeth of the rack in the extension direction of the rack is regarded as a reference surface; and it is assumed that a corresponding spacing between centers of every two adjacent tooth tips on the reference surface in the extension direction of the rack, i.e., a tooth spacing, is $t_1$, and a corresponding

spacing between tip ends of two adjacent locking plugs randomly selected from the plurality of locking plugs, close to the rack on the reference surface in the extension direction of the rack, i.e., a plug spacing, is $t_2$, where the plug spacing $t_2$ is a non-integer multiple of the tooth spacing $t_1$; and accordingly, when the sliding member moves to a position closest to the rack, the first tooth surface of at least one tooth and the second tooth surface of at least one tooth in the rack respectively abut against at least one locking plug of the plurality of locking plugs.

According to the foregoing technical solution, the flexibility of positional adjustment can be improved, and then stepless adjustment is achieved to some extent.

In a preferred embodiment, assuming the quantity of locking plugs in the plurality of locking plugs is denoted as x, $t_1$, $t_2$ and x satisfy the following Equation (1):

$$t_2 = \left(n \pm \frac{1}{x}\right) t_1 \ \text{Equation (1);}$$

where
n is a natural number greater than or equal to 1.

According to the foregoing technical solution, stepless adjustment and positioning of both the rack and the instrument can be achieved.

In a preferred embodiment, assuming in the extension direction of the rack, a is defined as a dimension of a narrowest part of the tip end closest to the rack, of a single locking plug of the plurality of locking plugs, and b is defined as a dimension of a widest part of the tooth slot, a and b satisfy Equation (2):

$$\frac{t_1}{x} \leq b - a \ \text{Equation (2).}$$

According to the foregoing technical solution, the locking plug can be made as thin as possible, and the opening of the tooth slot can be made as wide as possible, so that the locking plug can be more easily inserted into the tooth slot. This also allows two adjacent locking plugs to be inserted into the tooth slot of the rack simultaneously, thereby improving the utilization rate and locking efficiency of the locking plugs.

In a preferred embodiment, the sliding member is provided with a plurality of plug guide rails, and the plurality of plug guide rails and the plurality of locking plugs are of the same quantity, and are arranged in one-to-one correspondence; and accordingly, a single locking plug of the plurality of locking plugs is capable of moving toward or away from the rack via the corresponding plug guide rail, and the locking plug is limited by the plug guide rail on a section perpendicular to the extension direction of the locking plug.

According to the foregoing technical solution, the locking plug can only move radially toward or away from the rack, without causing unnecessary swinging.

In a preferred embodiment, the rack is in a curved shape, and when the bracket and the instrument are connected, the instrument is capable of being adjusted in position synchronously with the rack.

In a preferred embodiment, the rack is in an arc shape, and with a radial direction of a circumference of the rack being a radial direction of the adjustment and positioning device for instrument, a direction perpendicular to a radial section of the rack being an axial direction, and a circumferential direction of the rack being a circumferential direction of the rack being a circumferential direction of the rack, when the bracket and the instrument are connected, the instrument is capable of rotating synchronously with the rack around an axis extending axially from a center of the circumference of the rack.

According to the foregoing technical solution, the arc-shaped rack can drive the instrument to rotate around the center of the circumference of the rack, thereby adjusting the position and angle of the instrument.

In a preferred embodiment, the reference surface is an arc surface, and on the radial section of the rack, the tooth spacing $t_1$ is a corresponding circumferential arc length between the centers of every two adjacent tooth tips on the reference surface; and the plug spacing $t_2$ is a corresponding circumferential arc length between centers of the tip ends of two adjacent locking plugs randomly selected from the plurality of locking plugs, close to the rack, on the reference surface.

According to the foregoing technical solution, the arc length is equal to an included angle between two ends of the arc multiplied by a radius of the arc. Therefore, the tooth spacing $t_1$ and the plug spacing $t_2$, as the corresponding circumferential arc lengths on the reference surface, reflect information of the included angle between two adjacent teeth in the rack and information of the included angle between two adjacent locking plugs, respectively.

In a preferred embodiment, the tip end of a single locking plug of the plurality of locking plugs close to the rack is configured to be conical, trapezoidal, V-shaped, or spherical.

According to the foregoing technical solution, the tip end of the locking plug is more conveniently inserted into the tooth slot and fits better with the tooth surface of the rack, thereby improving the convenience and stability of locking.

In a preferred embodiment, the tip end of a single locking plug of the plurality of locking plugs close to the rack is configured to be conical, V-shaped, or trapezoidal, and an apex angle of a longitudinal section of the locking plug ranges from 20 to 150 degrees.

According to the foregoing technical solution, the apex angle of the longitudinal section of the tip end of the plug is not set too small, so as to prevent the tip end of the locking plug from being overly sharp, which could compromise the structural strength; and the apex angle is not set too large, so as to prevent the rack from applying excessive radial force on the locking plug, which could cause the locking plug to retreat and result in locking failure.

In a preferred embodiment, theadjustment and positioning device for instrument includes at least one roller/ball abutting against at least one side face of the bracket.

According to the foregoing technical solution, rolling friction can be generated between the rack and the roller/ball, thereby reducing the resistance during positional adjustment and minimizing abrasion on components.

In a preferred embodiment, at least a portion of the at least one roller/ball is located on the same side of the bracket as the rack, and a portion of the bracket on the same side as the rack is provided with a blank region without the rack, and for abutting against the roller/ball on the same side.

According to the foregoing technical solution, the roller/ball on the same side as the rack is set to abut against the blank region without the rack, thereby avoiding interference with and damage to the rack.

In a preferred embodiment, the sliding member abuts against the locking mechanism via a second elastic element at a position further back relative to a front end of the sliding member close to the rack in a movement direction of the sliding member, and the second elastic element allows a specified amount of deformation in the movement direction of the sliding member.

According to the foregoing technical solution, when the sliding member retracts to an unlocking position and the manual force is released, the sliding member can automatically move toward a locking position near the rack under the elastic force of the second elastic element, thereby achieving the purpose of automatic locking.

In a preferred option, when the sliding member moves to a locking position closest to the rack, an elastic force of the second elastic element is greater than or equal to a sum of elastic forces of the first elastic elements corresponding to each of the plurality of locking plugs abutting against the rack.

According to the foregoing technical solution, the sliding member can be prevented from retracting away from the rack under the acting force of the rack when in the locked state, thereby ensuring the locking firmness.

Theadjustment and positioning device for instrument in the foregoing embodiments of the present application can limit the positional adjustment of an instrument to a degree of freedom, while increasing the flexibility of positional adjustment, thereby achieving stepless adjustment to some extent. Additionally, theadjustment and positioning device for instrument is easy to process and cost-effective.

## BRIEF DESCRIPTION OF THE DRAWINGS

To describe the present application more clearly, the accompanying drawings of the specification are described and illustrated below. Apparently, the accompanying drawings in the following description merely illustrate certain aspects of some exemplary embodiments of the present application, and those of ordinary skill in the art may still derive other accompanying drawings from these accompanying drawings without creative efforts.

FIG. 1 is an external view illustrating an adjusting and positioning device and an instrument.

FIG. 2 is a schematic diagram illustrating an internal structure of the adjusting and positioning device.

FIG. 3 is a schematic diagram illustrating the locking state of the locking mechanism and the rack.

FIG. 4 is a schematic diagram illustrating the position of the tip end of the plug and the rack.

FIG. 5 is a partial enlarged view illustrating a straight rack.

FIG. 6 is a schematic diagram illustrating force analysis of the locking plug.

FIG. 7 is an enlarged view illustrating the locking mechanism.

FIG. 8 is a schematic diagram illustrating a sliding guide rail.

FIG. 9 is a schematic diagram illustrating a rolling guide rail.

FIG. 10 is a schematic diagram illustrating a structure of a button.

Text Description of Drawings:

100 adjusting and positioning device
200 instrument
1 bracket
11 bracket connecting portion
12 bracket sliding portion
13 rack
130 tooth tip
131. first tooth surface
132. second tooth surface
133 tooth slot
134 reference surface
14 blank region
2 positioning mechanism
21 rack guide rail
211 guide rail sliding surface
221 roller
22 side cover

222 roller
223 roller
224 roller
225 adjustment hole
226 adjustment screw
2210 pin
2220 pin
2230 pin
2240 pin
23 roller fixing base
3 locking mechanism
30 sliding member
31 first locking plug
310 tip end
3101 first plug bevel
3102 second plug bevel
3103 tip end face
3105 center line
3106 tangent line
32 second locking plug
320 tip end
3201 first plug bevel
3202 second plug bevel
3203 tip end face
33 guiding slot
34 locking base
35 spring
36 spring
37 spring
301 first sliding slot
302 second sliding slot
311 first plug guide rail
312 second plug guide rail
309 first protrusion portion
5 button
6 rotation block
60 rotation base
61 second protrusion portion
62 rotation cutting face
63 pin
8 clamp

## DETAILED DESCRIPTION OF EMBODIMENTS

Exemplary embodiments of the present disclosure will be described in detail below with reference to the accompanying drawings. The description of the exemplary embodiment is merely illustrative, and never constitutes any limitation to the present disclosure and application or use thereof. The present disclosure may, however, be implemented in many different forms and should not be limited to the embodiments set forth herein. These embodiments are provided so that the present disclosure is thorough and complete, and will fully convey the scope of the present disclosure to a person skilled in the art. It is to be noted that: unless otherwise stated, the relative arrangement, numerical expressions, and values of the components and steps described in these embodiments should be interpreted as merely exemplary, rather than restrictive.

"Comprising", "including" and similar words used in the present disclosure mean that an element appearing before the term includes elements listed after the term, without excluding any other elements.

Unless otherwise defined, all teams (including technical and scientific terms) used herein have the same meaning as those understood by a person of ordinary skill in the art to which the present disclosure belongs. It should also be further understood that terms, such as those defined in general dictionaries, should be interpreted as having a meaning that is consistent with their meaning in the context of the related art, and these terms should not be interpreted in an idealized or overly formal sense unless expressly so defined herein.

For components not described in detail in this section, specific parameters such as model numbers of components, as well as the interrelationships between components, and control circuits, they may be considered as technologies, methods, and equipment known to those skilled in the art. However, where appropriate, the technologies, methods, and equipment should be regarded as part of the specification.

(Overall Structure)

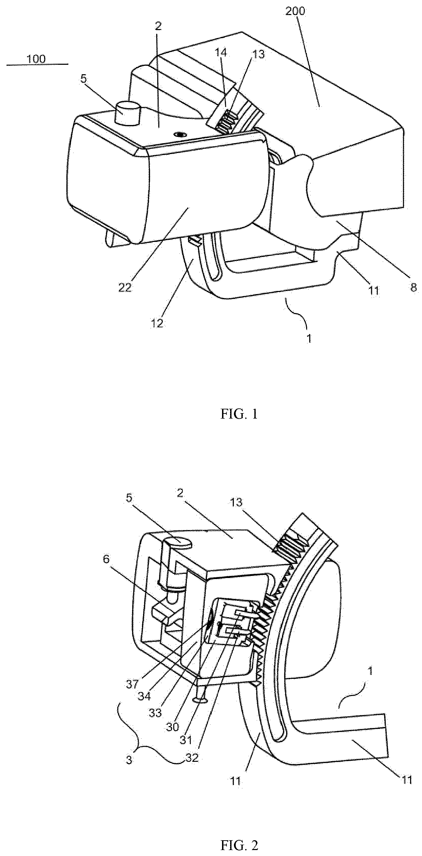

The structure of the adjustment and positioning device for instrument 100 of the present application will be described below with reference to FIG. 1. FIG. 1 is an external view illustrating an adjusting and positioning device 100 and an instrument 200.

As shown in FIG. 1, the adjusting and positioning device 100 includes a bracket 1, a positioning mechanism 2, and a locking mechanism 3 located inside the positioning mechanism 2, which is not shown in the figure. As an example, the bracket 1 includes a bracket connecting portion 11 and a bracket sliding portion 12 that are rigidly connected to each other. The instrument 200 is detachably connected to the bracket connecting portion 11 via a clamp 8. One side surface of the bracket sliding portion 12 is provided with a rack 13, and the bracket sliding portion 12 is capable of moving in a track defined by the positioning mechanism 2, thereby driving the instrument 200 for position adjustment. When a target position is reached, the locking mechanism 3 engages with the rack 13 to achieve a locked and fixed state.

During the operational process, since the precision of manual operation is difficult to control, the hands of an operator may involuntarily tremble, and the subject acted upon by the instrument 200, such as human tissue also exerts a certain amount of force upon the instrument 200, inevitably causing unintended positional and angular shifts to the instrument 200. This results in reduced positioning accuracy, increased manual operation difficulty, and raised risks of operational errors.

To address the foregoing problem, the present application provides a positioning mechanism 2. The positioning mechanism 2 remains stationary during the operational process, and the bracket sliding portion 12 moves only within one degree of freedom within the track defined by the positioning mechanism 2, so that the instrument 200 can only be adjusted within one degree of freedom after being fixed to the bracket 1. For example, when the bracket sliding portion 12 is linear and the rack 13 is straight, the bracket 1 can only move in a straight line in the extension direction of the rack 13. When the bracket sliding portion 12 is in an arc shape and the rack 13 is in an arc shape, the bracket 1 can only rotate synchronously with the rack 13. In this way, when there are deviations in manual operation, hand tremors, or external forces acting on the instrument 200, the instrument 200 can only move with the bracket 1 within one degree of freedom, thereby eliminating unnecessary disturbances, improving positioning accuracy and operational convenience, and reducing the risk of operational errors.

In addition, since there is a specific included angle or spacing between two adjacent teeth of a rack 13, in the prior art, a gear, a rack, a worm, and other components are typically used as the locking mechanism 3 to engage with the rack 13, thereby achieving the purpose of locking. The problem with this design is that: typically, there is also a specific angle or spacing between teeth or plugs in the locking mechanism 3 that engage with the rack 13, forcing the rack 13 to adjust the position only in fixed steps. For example, if the rack 13 is an arc-shaped rack and the included angle between two adjacent teeth is 3o, when the locking mechanism 3 adopts a gear to be in locking engagement with the rack 13, the rack 13 can generally only rotate in integer multiples of 3o to allow the gear of the locking mechanism 3 to engage with the rack 13. Otherwise, the rack 13 cannot be locked in the rotation direction. As a result, the instrument 200 cannot be adjusted in fine steps or adjusted steplessly, which increases the operational complexity and reduces the flexibility of positional adjustment.

However, a plurality of locking plugs that can be inserted into the rack 13 are adopted for the locking mechanism 3 of the present application. By properly setting the spacing or included angle between two adjacent locking plugs, it ensures that regardless of the rotation angle of the rack 13, there will always be a locking plug inserted into the rack 13 to lock the rack 13 in the rotation direction. This helps to achieve stepless adjustment to at least some degree, which will be discussed further later.

(Locking mechanism and rack)

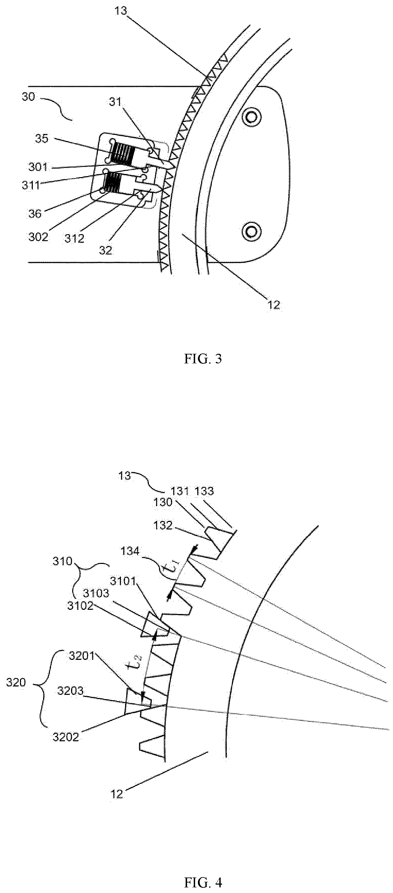

Next, the locking mechanism 3 will be described with reference to FIG. 2 and FIG. 3. FIG. 2 is a schematic diagram illustrating an internal structure of the adjusting and positioning device 100. FIG. 3 is a schematic diagram illustrating the locking state of the locking mechanism 3 and the rack 13.

Referring to FIG. 2, preferably, the locking mechanism 3 is disposed inside the positioning mechanism 2, and includes a locking base 34 fixedly engaged with the positioning mechanism 2, a guiding slot 33 provided on the locking base 34, a sliding member 30 accommodated in the guiding slot 33, and a plurality of locking plugs extending radially, such as the first locking plug 31 and the second locking plug 32 shown in the figure. In fact, the quantity x of a plurality of locking plugs is not limited to two; and can be greater than two. However, the quantity x of locking plugs cannot be 1, otherwise stepless adjustment cannot be achieved. For the sake of simplicity, only two locking plugs are used herein as an example for illustration.

Additionally, when the rack 13 is a straight-type rack, the normal direction is perpendicular to the side face of the bracket 1 where the rack 13 is located, and the extension direction of the rack 13 is a straight-line direction in which all the teeth of the rack 13 are arranged sequentially. The rack 13 can also be in a curved shape or arc shape. For example, when the rack is in an arc shape, the normal direction is the radial direction of the circumference of the rack 13, while the extension direction of the rack 13 is the circumferential direction of the circumference of the rack 13. For the sake of simplicity, unless otherwise specified, only the arc-shaped rack shown in the figure is used as an example for illustration.

At the same time, for ease of description, a radial direction of a circumference of the rack 13 is taken as a radial direction of the adjusting and positioning device 100, a direction perpendicular to a radial section of the rack 13 is taken as an axial direction of the adjusting and positioning device 100, and a circumferential direction of the circumference of the rack 13 is taken as a circumferential direction of the adjusting and positioning device 100. When the bracket 1 and the instrument 200 are connected, the instrument 200 rotates synchronously with the rack 13 around an axis extending axially from a center of the circumference of the rack 13, thereby achieving positional and angular adjustment.

As an example, the rack 13 is disposed on a radial outer surface of the bracket sliding portion 12. In practice, it can also be disposed on a radial inner surface of the bracket sliding portion 12, or on one end face of the bracket sliding portion 12 facing the axial side. No specific limitation is made herein; for illustration, the rack 13 is shown as being disposed on the radial outer side of the bracket sliding portion 12.

Still referring to FIG. 2, preferably, the guiding slot 33 is a straight slot located on the radial outer side of the rack 13 and extending radially. The sliding member 30 is embedded in the guiding slot 33 and can only move in a linear direction, either away from or toward the rack 13 in the radial direction of the rack 13.

Referring to FIG. 3, preferably, the sliding member 30 is provided with a first sliding slot 301 for receiving the first locking plug 31 and a second sliding slot 302 for receiving the second locking plug 32. The front ends of the first sliding slot 301 and the second sliding slot 302 close to the rack 13 are also provided a first plug guide rail 311 and a second plug guide rail 312, which are through-hole types and serve as plug guide rails. The first locking plug 31, first sliding slot 301, and first plug guide rail 311, as well as the second locking plug 32, second sliding slot 302, and second plug guide rail 312 all extend radially.

In addition, the first locking plug 31 and the second locking plug 32 are connected to the sliding member 30 at rear positions such as rear ends that are far away from the rack 13 in the radial direction via springs 35 and 36 as first elastic elements, respectively.

As a result, during the locking process, the sliding member 30 moves in the extension direction of the guiding slot 33, i.e., radial direction, toward the rack 13, thereby pushing the first locking plug 31 and the second locking plug 32 to move toward the rack 13 along the first plug guide rail 311 and the second plug guide rail 312 via the springs 35 and 36 respectively, and driving the first locking plug 31 and the second locking plug 32 to partially extend to a position between the sliding member 30 and the rack 13. When the sliding member 30 moves to the position closest to the rack 13, the tip ends of the first locking plug 31 and the second locking plug 32 close to the rack 13 abut against the rack 13 and limit rotational movement of the rack 13 in the circumferential direction, thereby achieving the purpose of locking the bracket 1. When unlocking is required, the sliding member 30 retracts radially away from the rack 13, and the first locking plug 31 and the second locking plug 32 are driven to retract radially via springs 35 and 36, and disengage from the rack 13, thereby achieving the unlocking purpose, and conducting positional adjustment on the bracket 1.

In this embodiment, the first locking plug 31 and the second locking plug 32 can only move linearly away from/close to the rack 13 in the radial direction under the limit of the first plug guide rail 311 and the second plug guide rail 312. That is, the first locking plug 31 and the second locking plug 32 are respectively limited by the first plug guide rail 311 and the second plug guide rail 312 in the direction perpendicular to the extension direction thereof.

In addition, since rear positions such as rear ends of the first locking plug 31 and the second locking plug 32 away from the rack 13 are connected to the sliding member 30 via springs 35 and 36 respectively, the first locking plug 31 and the second locking plug 32 can, when in contact with the rack 13, retract radially away from the rack 13 under the force exerted by the rack 13. In other words, the first locking plug 31 and the second locking plug 32 can float by a certain distance radially, respectively.

It can be understood that the locking mechanism 3 matched with the rack 13, is correspondingly positioned on the same side of the bracket sliding portion 12 as the rack 13. For example, when the rack 13 is provided on a radial inner surface or an axial side end surface of the bracket sliding portion 12, the locking mechanism 3 is also provided on the radial inner side or the axial side end surface of the bracket sliding portion 12, and details are not described herein.

It should be noted that the rack 13 in the present application is a broadly defined rack and is not limited to traditional rack types such as straight racks or curved racks. For example, conical recesses arranged in sequence can be dug on one side surface of the bracket sliding portion 12, and the locking plug of the locking mechanism 3, such as the first locking plug 31 and the second locking plug 32, can be inserted into the conical recesses respectively, thereby achieving the technical effect of the present application. At this point, longitudinal sections of the consecutively arranged conical recesses are equivalent to the rack 13 sequentially arranged, and details are not described herein.

(Tip ends of plugs and teeth)

Next, tip ends of the locking plugs and teeth of the rack 13 are described with reference to FIG. 4. FIG. 4 is a schematic diagram illustrating the position of the tip end of the plug and the rack 13.

Referring to FIG. 4, on the radial section of the rack 13, the tooth tip 130 is provided at the end of each tooth of the rack 13 that is located on the radial outer side and close to the sliding portion 30. In the circumferential direction of the circumference of the rack 13, each tooth further includes a first tooth surface 131 and a second tooth surface 132 which are distributed on both sides of the tooth tip 130, extend from the tooth tip 130 toward the radial inner side away from the sliding portion 30, and tilt toward opposite sides of the normal line, respectively. At the same time, a gap, i.e., tooth slot 133, is further provided between two adjacent teeth. The tooth tip 130 shown in the figure is a flat end surface perpendicular to the radial direction. In practice, it can also be a curved surface or a tip formed by the intersection of the first tooth surface 131 and the second tooth surface 132. The first tooth surface 131 and the second tooth surface 132 can either be smooth slopes or take the form of an involute gear tooth profile. No specific limitations are made herein.

FIG. 4 also shows the tip end 310 of the first locking plug 31, and the tip end 320 of the second locking plug 32. For the sake of simplicity, the remaining parts of the first locking plug 31 and the second locking plug 32 are not shown in the figure.

Preferably, the tip end 310 of the first locking plug 31, and the tip end 320 of the second locking plug 32 can be configured to be conical, V-shaped, trapezoidal, or spherical dome type. Taking the trapezoidal shape shown in FIG. 4 as an example, on the radial section, the position of the tip end 310 of the first locking plug 31 closest to the rack 13 in the radial direction is provided with a tip end face 3103 perpendicular to the radial direction, and a first plug bevel 3101 and a second plug bevel 3102 extending from the tip end face 3103 away from the rack 13, which are symmetrically distributed on both sides of the tip end face 3103 in the circumferential direction, and have a specified included angle therebetween.

Similarly, on the radial section, the position of the tip end 320 of the second locking plug 32 closest to the rack 13 in the radial direction is provided with a tip end face 3203 perpendicular to the radial direction, and a first plug bevel 3201 and a second plug bevel 3202 extending from the tip end face 3203 away from the rack 13, which are symmetrically distributed on both sides of the tip end face 3203 in the circumferential direction, and have a specified included angle therebetween.

Under the locking state of the rack 13, at least one of the tip end 310 of the first locking plug 31 and the tip end 320 of the second locking plug 32 is inserted into the tooth slot 133. The first plug bevel (3101, 3201) abuts against the second tooth surface 132 of a tooth in the rack 13, while the second plug bevel (3102, 3202) abuts against the first tooth surface 131 of a tooth in the rack 13, thereby limiting the rack 13 from rotating in the circumferential direction, and achieving the purpose of locking.

(Plug spacing and tooth spacing)

Next, the plug spacing $t_2$ of the locking plug and the tooth spacing $t_1$ of the rack 13 are described with reference to FIG. 4.

Referring to FIG. 4, a surface that is tangent to tooth tips 130 of all the teeth of the rack 13 in the circumferential direction is taken as a reference surface 134. When the rack 13 is an arc-shaped rack, the reference surface 134 is an arc surface which is along the circumference of the rack 13, and tangent to the tooth tip 130 of each tooth. As an example, on the radial section of the rack 13, the tooth spacing $t_1$ is defined as a corresponding circumferential arc length between the centers of every two adjacent tooth tips 130 of the rack 13 on the reference surface 134; and the plug spacing $t_2$ is defined as a corresponding circumferential arc length between the center of the tip end face 3103 of the tip end 310 of the first locking plug 31 and the center of the tip end face 3203 of the tip end 320 of the first locking plug 32 on the reference surface 134.

It can be understood that the tooth spacing $t_1$ is defined as the included angle between the centers of two adjacent tooth tips 130 and the center of the circumference of the reference surface 134, respectively, multiplied by the corresponding radius on the circumference. The plug spacing $t_2$ is defined as the included angle between the center of the tip end face 3103 and the center of the tip end face 3203, respectively, and the center of the circumference of the reference surface 134, multiplied by the corresponding radius on the circumference.

Certainly, if the tip end (310, 320) is configured to be conical, V-shaped, or spherical dome type, the plug spacing $t_2$ is the circumferential arc length on the reference surface 134, corresponding to the position of the tip ends (310, 320) closest to the rack 13 in the radial direction, such as the cone tip, V-tip, or the apex of the dome. Details are not described herein.

Additionally, if the rack 13 is a straight rack, the reference surface 134 is a plane parallel to the rack 13. On the radial section, the tooth spacing $t_1$ is defined as a corresponding linear distance between centers of two adjacent tooth tips 130 on the reference surface 134, and the plug spacing $t_2$ is defined as a corresponding linear distance between centers of tip ends (310, 320) of two adjacent locking plugs on the reference surface 134. However, for the sake of simplicity, only the arc-shaped rack is used herein as an example for illustration.

Preferably, the plug spacing $t_2$ is a non-integer multiple of the tooth spacing $t_1$. As mentioned above, when a mechanism such as a gear, a rack, or a worm is in locking engagement with the rack 13, the adjustment step of the rack 13 can only be an integer multiple of the tooth spacing $t_1$. However, in the technical solution of the present application, the locking plugs of the locking mechanism 3, such as the first locking plug 31 and the second locking plug 32, can float radially respectively, and the plug spacing $t_2$ is a non-integer multiple of the tooth spacing $t_1$, which allows for more diverse adjustment step options for the rack 13. For example, when the adjustment step does not satisfy an integer multiple of the tooth spacing $t_1$, all or part of the plurality of floating locking plugs can still be inserted into different depth positions of one or more tooth slots 133 and respectively abut against the first tooth surface 131 or the second tooth surface 132. This solves the problem of ineffective locking engagement in traditional technologies under such circumstances, achieves locking and positioning of the rack 13, and improves the flexibility of positional adjustment, thereby achieving stepless adjustment to a certain extent.

Furthermore, the tooth spacing $t_1$ and the plug spacing $t_2$ satisfy the following Equation (1):

$$t_2 = \left(n \pm \frac{1}{x}\right) t_1 \quad \text{Equation (1);}$$

where
x denotes the quantity of locking plugs, and n is a natural number greater than or equal to 1.

In this embodiment, the quantity x of locking plugs is 2, and the plug spacing $t_2$ between the first locking plug 31 and the second locking plug 32 is as follows:

$$t_2 = \left(n \pm \frac{1}{2}\right) t_1.$$

That is, the plug spacing $t_2$ is an integer multiple of half the tooth spacing $t_1$, such as 0.5 times, 1.5 times, or 2.5 times as shown in FIG. 3. At this point, regardless of the rotation angle of the rack 13, when the sliding member 30 moves to the locking position closest to the rack 13, at least one of the first locking plug 31 and the second locking plug 32 is inserted into the rack 13 and engages with one of the tooth surfaces, such as the first tooth surface 131, and the other locking plug is also inserted into the rack 13 and engages with the other tooth surface, such as the second tooth surface 132. Alternatively, as shown in FIG. 3, one locking plug is inserted into the bottom of the tooth slot 133, while the other locking plug abuts against the tooth tip 130, thereby limiting the rotation of the rack 13 in the circumferential direction.

Similarly, when the quantity x of locking plugs is greater, such as 3 or 4, as long as the tooth spacing $t_1$ and the plug spacing $t_2$ satisfy Equation (1), regardless of the rotation angle of the rack 13, when the sliding member 30 moves to the locking position closest to the rack 13, the first tooth surface 131 of at least one tooth and the second tooth surface 132 of at least one tooth of the rack 13 each abut against at least one locking plug, thereby limiting the rotation of the rack 13 in the circumferential direction.

It can be understood that, at this point, the first tooth surface 131 and the second tooth surface 132 abutting against the locking plugs, can either be located on the same tooth or on different teeth. Regarding the locking plugs, one locking plug may be inserted into the bottom of the tooth slot 133, and abut against the first tooth surface 131 and the second tooth surface 132 on two sides, respectively. Alternatively, a plurality of locking plugs may be inserted into different tooth slots 133 respectively, and abut against a plurality of different first tooth surfaces 131 and second tooth surfaces 132, respectively. For example, the first locking plug 31 is inserted into one tooth slot 133 and abuts against the first tooth surface 131, while the second locking plug 32 is inserted into another tooth slot 133 and abuts against the second tooth surface 132.

It should be noted that, according to Equation (1), when the quantity x of locking plugs is greater than 2, due to the existence of a natural number n greater than or equal to 1, the plug spacing $t_2$ between two adjacent locking plugs is not necessarily equal, that is, the plurality of locking plugs may not be distributed at equal intervals. For example, when there are 4 locking plugs, the plug spacing $t_2$ between two adjacent locking plugs can be as follows:

$$t_2 = \left(1 \pm \frac{1}{4}\right) t_1.$$

At this point, n = 1. The plug spacing $t_2$ between the other two adjacent locking plugs can be as follows:

$$t_2 = \left(2 \pm \frac{1}{4}\right) t_1.$$

At this point, n = 2. That is, the specific size of the plug spacing $t_2$ between different adjacent locking plugs is determined by the value of n. Certainly, preferably, the plurality of locking plugs of the present application are arranged at equal intervals. That is, the plug spacing $t_2$ between any two adjacent locking plugs is consistent.

It can be understood that, when the rack 13 is a straight rack, the rack 13 is no longer adjusted by rotation, but rather by translation in the extension direction of the rack 13. Similarly, as long as the tooth spacing $t_1$ and the plug spacing $t_2$ satisfy Equation (1), regardless of the movement distance of the rack 13, when the sliding member 30 moves to the locking position closest to the rack 13, the first tooth surface 131 of at least one tooth and the second tooth surface 132 of at least one tooth of the rack 13 each abut against at least one locking plug, thereby limiting the movement of the rack 13. Details are not repeated herein.

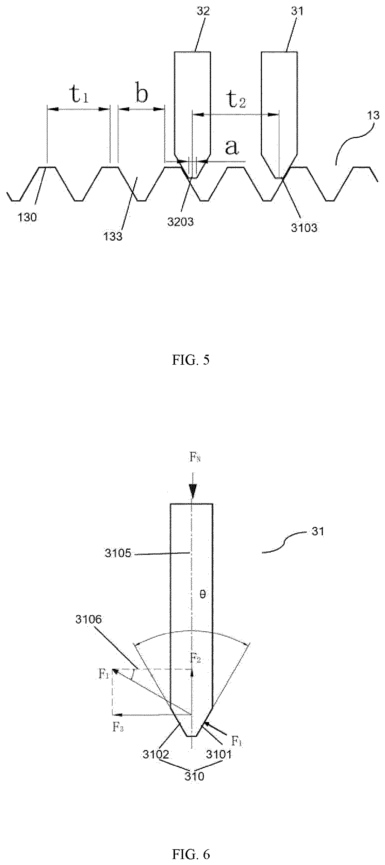

Next, the relationship between the plug spacing $t_2$ and the tooth spacing $t_1$ is described with reference to FIG. 5. FIG. 5 is a partial enlarged view of a rack 13 as a straight rack.

As shown in FIG. 5, on the radial section, a is defined as the dimension of a narrowest part of the tip end (310, 320) close to the rack 13, of the first locking plug 31 and the second locking plug 32. As shown in FIG. 5, a refers to the dimension of the tip end face 3103 of the first locking plug 31 and the dimension of the tip end face 3203 of the second locking plug 32. It can be understood that if the tip ends (310, 320) are configured to be conical, V-shaped, or spherical dome type, a is zero. Details are not described herein.

The dimension of the widest part of the tooth slot 133 is b. Under the locking state of the rack 13, for two adjacent locking plugs to be simultaneously inserted into the rack 13, the following Equation (2) needs to be satisfied:

$$\frac{t_1}{x} \leq b - a \quad \text{Equation (2).}$$

The derivation process of Equation (2) is described using the straight rack shown in FIG. 5 as an example.

As can be intuitively seen from FIG. 5, when two adjacent locking plugs, such as the first locking plug 31 and the second locking plug 32, are simultaneously inserted into two adjacent tooth slots 133, the tooth spacing $t_1$ and the plug spacing $t_2$ need to satisfy the following relation:

$$a + t_2 \leq b + t_1 \quad \text{Equation (3).}$$

Combining with Equation (1), since the first locking plug 31 and the second locking plug 32 are simultaneously inserted into two adjacent tooth slots 133, the plug spacing $t_2$ is less than two times the tooth spacing $t_1$, and greater than 1 times the tooth spacing $t_1$.

Therefore, by setting n = 1 in Equation (1) and disregarding $n - \frac{1}{x}$ inside the parentheses of Equation (1), it is obtained that:

$$t_2 = \left(1 + \frac{1}{x}\right) \text{ Equation (4)}.$$

In addition, if the first locking plug 31 and the second locking plug 32 are simultaneously inserted into the same tooth slot 133, it can be intuitively obtained from FIG. 5 that $a + t_2 \leq b$. At this point, the relationship among a, b and $t_2$ still satisfies Equation (3). However, since the two locking plugs are inserted into the same tooth slot 133, the plug spacing $t_2$ is less than one times the tooth spacing $t_1$ in this case.

Therefore, by setting n = 1 in Equation (1) and disregarding $n + \frac{1}{x}$ inside the parentheses of Equation (1), it is obtained that:

$$t_2 = \left(1 - \frac{1}{x}\right) t_1 \text{ Equation (6)}.$$

By combining Equation (4) and Equation (6), it can be obtained that: $t_2 = \left(1 \pm \frac{1}{x}\right) t_1$ Equation (7).

Next, considering the case where n is a larger value, i.e., the plug spacing $t_2$ between two adjacent locking plugs is further increased, since in Equation (1), n is defined to be a natural number greater than or equal to 1, when the plug spacing $t_2$ is increased, it can only be increased by an integer multiple of the tooth spacing $t_1$. For example, the first locking plug 31 is moved by an integer multiple of the tooth spacing $t_1$ away from the second locking plug 32.

Still referring to FIG. 5, assuming the first locking plug 31 is moved away from the second locking plug 32 by (n-1) times the tooth spacing t, combining with Equation (3), it can be obtained that: $a + t_2 + (n - 1) t_1 \leq b + t_1 + (n - 1) t_1$.

The following can be obtained by substituting Equation (7):

$$a + \left(1 \pm \frac{1}{x}\right) t_1 + (n - 1) t_1 \leq b + t_1 + (n - 1) t_1$$

.

Further, the following can be derived: $a + \left(n \pm \frac{1}{x}\right) t_1 \leq b + n t_1$.

Combining like terms yields the following: $\left(\pm \frac{1}{x}\right) t_1 \leq b - a$.

Since the distance cannot be negative, taking the absolute value of $\left(\pm \frac{1}{x}\right) t_1$ $t_1$ gives Equation (2):

$$\frac{t_1}{x} \leq b - a \text{ Equation (2)}.$$

The above is illustrated by using the straight rack as an example. Since a straight line can be considered as a segment of an infinite circumference, similarly, in the case where the rack 13 is an arc-shaped rack, the tooth spacing $t_1$ and the plug spacing $t_2$ still need to satisfy Equation (2), so that any two adjacent locking plugs can be simultaneously inserted into the tooth slots 133 of the rack 13. However, in this case, b represents the corresponding arc length of the widest part of the tooth slot 133 in the radial direction on the circumference of the tooth tip 130.

When the tooth spacing $t_1$ and the plug spacing $t_2$ satisfy the Equation (2), it usually indicates that the dimension of the part of each locking plug inserted into the tooth slot 133 on the radial section should be as small as possible, i.e., not too thick. On the other hand, the dimension of the widest part of the tooth slot 133 on the radial section should be as large as possible, indicating that an opening of the tooth slot 133 should not be too narrow. As a preferred solution, it not only allows each locking plug to be more easily inserted into the tooth slot 133, but also allows any two adjacent locking plugs to be simultaneously inserted into one tooth slot or more different tooth slots 133. This improves the utilization rate of the locking plugs, enhances the locking efficiency and firmness, and also relatively reduces the quantity of locking plugs needed.

(Force analysis of the locking plug)

Next, force exerted on the locking plug is described with reference to FIG. 6. FIG. 6 is a schematic diagram illustrating force analysis of the locking plug.

Referring to FIG. 6, taking the first locking plug 31 as an example, on the radial section of the first locking plug, the tip end 310 is of a trapezoidal structure, with an included angle θ formed between the first plug bevel 3101 and the second plug bevel 3102. When the first locking plug 31 is inserted into the tooth slot 133, it may either be the first plug bevel 3101 that abuts against the second tooth surface 132, or the second plug bevel 3102 that abuts against the first tooth surface 131. For the sake of simplicity, only the case where the first plug slant edge 3101 abuts against the second tooth surface 132 is used as an example for illustration.

On the radial section, when the first plug bevel 3101 abuts against the second tooth surface 132, the second tooth surface 132 usually exerts a force $F_1$ on the first plug bevel 3101 in a direction perpendicular to the first plug bevel 3101, and the force $F_1$ has a component force $F_2$ in the direction of the center line 3105 extending radially and passing through the center of the tip end 310, where the component force $F_2$ is directed away from the rack 13; and there is a component force $F_3$ in the direction of the tangent line 3106 perpendicular to the center line 3105, and the component force $F_3$ is directed toward the second plug bevel 3102.

Since the first plug bevel 3101 and the second plug bevel 3102 are symmetrically distributed along a center line 3105, an included angle between the first plug bevel 3101 and the center line 3105 is $\frac{1}{2}\theta$. Furthermore, since $F_1$ is perpendicular to the first plug bevel 3101, the included angle between $F_1$ and the center line 3105 is $90° - \frac{1}{2}\theta$. In the meanwhile, since the tangent line 3106 is perpendicular to the center line 3105, it can be obtained that the included angle between $F_1$ and the tangent line 3106 is also $\frac{1}{2}\theta$. Thus:

$$F_2 = F_1 \sin\left(\frac{1}{2}\theta\right) \text{ Equation (8)};$$

$$F_3 = F_1 \cos\left(\frac{1}{2}\theta\right) \text{ Equation (9)}.$$

Additionally, in this embodiment, the rear end of the first locking plug 31 also abuts against a spring 35 acting as a first elastic element, and the spring 35 exerts a force Fn on the first locking plug 31. Under the locking state of the rack 13, Fn is directed toward the rack 13 along the center line 3105.

In summary, the first locking plug 31 is subject to opposite forces Fn and $F_2$ in the direction of the center line 3105, and a force $F_3$ in the direction of the tangent line 3106. As can be seen from Equation (8) and Equation (9), when the force $F_1$ exerted on the second tooth surface 132 is constant, the magnitudes of $F_2$ and $F_3$ are related to the included angle θ between the first plug bevel 3101 and the second plug bevel 3102. The force Fn is related to the elastic coefficient and deformation amount of the spring 35 on the center line 3105.

Specifically, as can be seen from Equation (8), if θ is too large, $F_2$ will also increase. When θ reaches a certain value, $F_2$ will be greater than Fn. In such case, the first locking plug 31 may move along the center line 3105 away from the rack 13, thus retracting from the tooth slot 133. This results in the risk that the rack 13 cannot be securely locked in position. As can be seen from Equation (9), if θ is too small, $F_3$ will be too large. When $F_3$ reaches a certain value, the first locking plug 31 will be pressed in the direction of the tangent line 3106 to fit more tightly with the inner wall of the first plug guide rail 311 through which the first locking plug 31 passes. In this way, the frictional force between the two is increased, so that the first locking plug 31 is less prone to be pulled out from the tooth slot 133 when the rack 13 is unlocked.

Additionally, if θ is too small, the tip end 310 becomes sharper. As a result, when the tip end 310 stretches into the tooth slot 133, neither the first plug bevel 3101 nor the second plug bevel 3102 can abut against the second tooth surface 132 and the first tooth surface 131. Instead, only the tiny top of the tip end 310 closest to the rack 13 abuts against the rack 13. This leads to insufficient mechanical strength, making the structure prone to damage and failure to lock. The problem is more pronounced when the tip end 310 has a conical or V-shaped structure.

Preferably, the included angle θ between the first plug bevel 3101 and the second plug bevel 3102 ranges from 20 degrees to 150 degrees. Within this range, the locking plug is less likely to retract under the locking state, and meanwhile, certain mechanical strength is ensured, thereby achieving locking stability and firmness.

(Second elastic element)

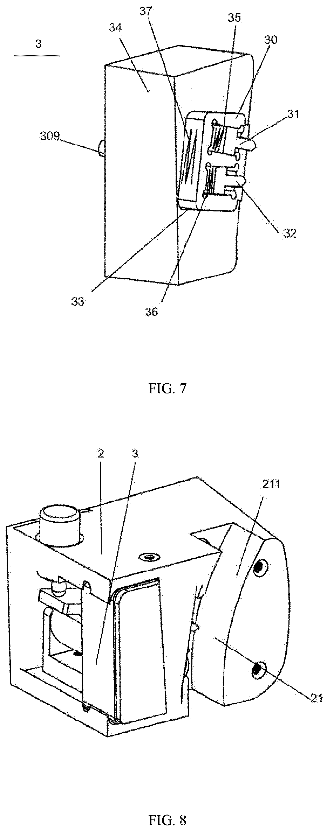

Next, the second elastic element is described with reference to FIG. 7. FIG. 7 is an enlarged view of the locking mechanism 3.

Referring to FIG. 7, the sliding member 30 is provided in the guiding slot 33 of the locking base 34. Preferably, the rear position such as a rear end of the sliding member 30 away from the rack 13 abuts against the locking base 34 via the spring 37 which serves as a second elastic element.

During the unlocking of the rack 13, the sliding member 30 retracts away from the rack 13 along the guiding slot 33 extending radially under the action of an external force, and the spring 37 is compressed. At the same time, the spring 35 and the spring 36 respectively pull the first locking plug 31 and the second locking plug 32 out of the tooth slot 133, thereby unlocking the rack 13.

When the rack 13 needs to be locked, the external force applied to the sliding member 30 is released, and the sliding member 30 moves toward the rack 13 along the radially extending guiding slot 33 under the elastic force of the spring 37. At this point, the spring 35 and the spring 36 are compressed, and the first locking plug 31 and the second locking plug 32 are pushed to move radially toward the rack 13. When the sliding member 30 moves to the position closest to the rack 13, the locking of the rack 13 is achieved.

It is to be noted that when a plurality of locking plugs are inserted into the rack 13, each of the locking plugs can independently move radially close to or away from the rack 13 due to the presence of the first elastic elements, such as the spring 35 and the spring 36. In other words, under the locking state of the rack 13, some locking plugs are inserted deeper into the tooth slot 133, even reaching the bottom of the tooth slot 133, thus being closer to the center of the circumference of the rack 13; while other locking plugs are inserted shallowly into the tooth slot 133, or merely abut against the tooth tip 130 without stretching into the tooth slot 133, thus being farther from the center of the circumference.

Taking the first locking plug 31 and the second locking plug 32 in FIG. 3 as an example, at this moment, the second locking plug 32 is not inserted into the tooth slot 133, but instead abuts against the tooth tip 130. The corresponding spring 36 is compressed more tightly, thereby providing a greater radial elastic force that pushes the sliding member 30 to retract away from the rack 13. In the meanwhile, the first locking plug 31 is inserted to the bottom of the tooth slot 133, and the corresponding spring 35 is less compressed, resulting in a smaller radial spring force that pushes the sliding member 30 to retract away from the rack 13.

In contrast, when the sliding member 30 moves to a locking position closest to the rack 13, the spring 37, as the second elastic element, remains in a compressed state, and provides a radial elastic force that pushes the sliding member 30 to move toward the rack 13. The elastic force should be greater than or equal to the sum of the radial elastic forces exerted on the sliding member 30 by the spring 35 and the spring 36, ensuring that the sliding member 30 is securely pressed against the locking position closest to the rack 13 and is not pushed away by the spring 35 and the spring 36.

(Rack guide rail)

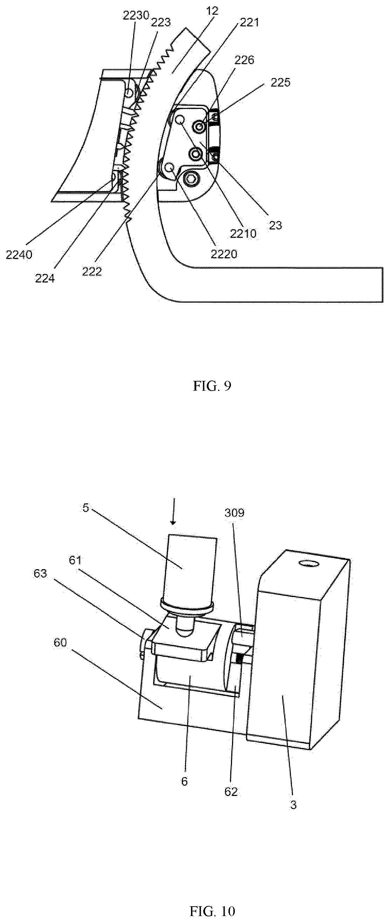

Next, the rack guide rail 21 is described in detail with reference to FIG. 8 and FIG. 9. FIG. 8 is a schematic diagram of a sliding guide rail, and FIG. 9 is a schematic diagram of a rolling guide rail.

Referring to FIG. 8, as an example, the positioning mechanism 2 includes a rack guide rail 21 matched with the rack 13. The rack guide rail 21 includes a guide rail sliding surface 211 abutting against the radial inner surface of the bracket 1. Under the working state, the bracket 1 is mounted within the rack guide rail 21, and the radial inner surface of the bracket 1 can slide along the guide rail sliding surface 211, thereby achieving positional adjustment.

In this embodiment, the rack guide rail 21 is formed as a recess in the axial direction on an axial side end surface of the positioning mechanism 2. The dimension and shape of the radial section of the rack guide rail 21 are matched with the dimension and shape of the radial section of the bracket 1. This configuration allows the bracket 1 to be readily mounted into the rack guide rail 21. Subsequently, the side cover 22 as shown in FIG. 1 is fixedly mounted on the axial outer side of the rack guide rail 21, so that the bracket 1 is constrained from moving in the axial direction, and can only perform rotational movement within one degree of freedom along the track defined by the rack guide rail 21. It can be understood that, if the rack 13 is a straight rack, the rack guide rail 21 is a linear guide rail matched with the rack. In this case, the rack 13 can only perform a translational movement within one degree of freedom in the rack guide rail 21.

Since the radial inner surface of the bracket 1 is in surface contact with the guide rail sliding surface 211, there is sliding friction between the two in the sliding process. This may lead to increased resistance in adjusting the position of the bracket 1 and also cause easy occurrence of abrasion, which affects the service life.

To address this, as a preferred solution, as shown in FIG. 9, on the radial section, the roller 221 and roller 222 are arranged circumferentially and abut against the radial inner surface of the bracket 1. As a result, the bracket 1 can move along an arc track formed by the roller 221 and roller 222. That is, the roller 221 and roller 222 replace the guide rail sliding surface 211, so that sliding friction is transformed into rolling friction, which not only reduces effort but also minimizes the abrasion of components. It can be understood that the roller 221 and roller 222 can be of various types, such as rollers or balls, as long as they are capable of rolling. No specific limitations are made herein.

At the same time, the quantity of rollers/balls is not limited to two; it can be one or more. Only two rollers/balls are used herein as an example for illustration. The rollers/balls are not limited to being located on a radial side of the bracket 1 opposite to the rack 13. Since the bracket sliding portion 12 has four sides abutting against the rack guide rail 21, the rollers/balls can be disposed on any side or a plurality of sides of the bracket sliding portion 12 abutting against the rack guide rail 21.

Still referring to FIG. 9, preferably, the radial outer side of the rack 13 is also provided with rollers/balls, such as the roller 223 and roller 224 arranged in the circumferential direction. Since the roller 223 and roller 224 should not directly abut against the rack 13, as a preferred solution, as shown in FIG. 1, the bracket 1 is provided with a blank region 14 that extends axially from one side of the rack 13 in the axial direction, and is not provided with the rack 13. The roller 223 and roller 224 abut against the blank region 14 separately, thereby being axially offset from the rack 13 to avoid mutual interference.

In this embodiment, by providing a plurality of rollers on both the radial inner side and radial outer side of the bracket sliding portion 12, a more stable clamping force is applied to the bracket 1. This makes the movement of the bracket 1 more stable, preventing unnecessary wobbling, which in turn facilitates locking. As an example, FIG. 9 also shows pin 2210, pin 2220, pin 2230, and pin 2240, which extend axially and are used to fix the roller 221, roller 222, roller 223, and roller 224, respectively. A bearing can further be mounted between each pin and the corresponding roller. Since this is not the focus of the present invention, details are not described herein.

However, during the fitting process of the bracket 1 and the plurality of rollers, there will inevitably be fitting tolerances between them. That is, gaps that could lead to wobbling may be present between the bracket 1 and the plurality of rollers. To address this, as a preferred solution, a roller fixing base 23 for position adjustment is provided.

Specifically, the roller 221 and roller 222 are fixed to the roller fixing base 23 through the pin 2210 and pin 2220, respectively. The roller fixing base 23 is provided with an adjustment hole 225 that is matched an adjustment screw 226. After passing through the adjustment hole 225, the adjustment screw 226 is screwed into the positioning mechanism 2, and tightened to achieve a fixed connection between the roller fixing base 23 and the positioning mechanism 2.

The adjustment screw 226 is provided with a relatively thick screw base and a thinner screw body that extends perpendicular to the screw base. In this embodiment, the outer diameter of the adjustment hole 225 is greater than the outer diameter of the screw body, and smaller than the outer diameter of the screw base. Thus, when the adjustment screw 226 is not yet tightened, there is a certain gap between the screw body of the adjustment screw 226 and the adjustment hole 225, allowing the roller fixing base 23 to achieve position adjustment relative to the positioning mechanism 2 in the direction toward or away from the rack 13. As a result, the roller 221 and roller 222 achieve position adjustment along with the roller fixing base 23. Once the roller 221 and roller 222 closely fit the radial inner surface of the bracket 1 to make the bracket 1 securely clamped by the plurality of rollers, the adjustment screw 226 is tightened. At this point, the screw base with the larger outer diameter fastens the roller fixing base 23 onto the positioning mechanism 2.

Through the design of position adjustment, the fitting tolerance can be overcome. This not only lowers machining precision of components, but also achieves zero-gap contact between the bracket 1 and a plurality bearing. As a result, the locking is more stable, and the rotation is also more flexible.

(Button and rotation block)

Next, the button 5 is described with reference to FIG. 10. FIG. 10 is a schematic diagram illustrating a structure of a button 5.

Referring to FIG. 10, as an example, the adjusting and positioning device 100 also includes a button 5 as a pressing member, a rotation block 6, and a rotation base 60 fixed to the positioning mechanism 2. The rotation block 6 is fixed to the rotation base 60 via an axially extending pin 63, allowing itself to rotate around a rotational axis of the pin 63.

The rotation block (6) is provided with a second protrusion portion (61) protruding outward from an outer surface thereof which is generally parallel to the pin 63. One end of the button 5 close to the rotation block 6 abuts against the second protrusion portion 61. In the meanwhile, the rotation block 6 is adjacent to the sliding member 30 in the axial direction. The sliding member 30 is provided with a first protrusion portion 309 extending axially from the side close to the rotation block 6. One side of the rotation block 6 close to the sliding member 30 is provided with a rotation cutting face 62 that is generally parallel to the extension direction of the first protrusion portion 309 and abuts against the first protrusion portion 309.

Thus when the button 5 is pressed manually, the button 5 can move in the direction of the arrow in the figure, and pushes the rotation block 6 to rotate around the rotational axis of the pin 63 via the second protrusion portion 61. When the rotation block 6 rotates, the rotation cutting face 62 pushes the first protrusion portion 309, which in turn drives the sliding member 30 to move radially away from the rack 13. When the manual pressure is released, the sliding member 30 moves in the direction toward the rack 13 under the elastic force of the second elastic element, i.e., the spring 37.

In other words, during the unlocking of the rack 13, normally the sliding member 30 needs to be manually pulled to move radially away from the rack 13. However, in this embodiment, it is only necessary to press the button 5, which transmits force to cause the sliding member 30 to move accordingly. An additional step of force transmission is added, while the direction of the force applied is changed. This is because pressing with fingers from top to bottom is more convenient than pulling from front to back, thus increasing the ease of the unlocking operation.

In summary, the present application achieves position adjusting and locking on the instrument 200 through the cooperation of a plurality of locking plugs with the rack 13; and restricts the movement of the instrument 200 to be within one degree of freedom through the cooperation of the rack 13 and the rack guide rail 21. Particularly, by reasonably designing the plug spacing $t_2$ between two adjacent locking plugs, it is ensured that when the sliding member 30 moves to the locking position closest to the rack 13, the first tooth surface 131 of at least one tooth and the second tooth surface 132 of at least one tooth of the rack 13 each abut against at least one locking plug, thereby achieving stepless adjustment on the rack 13 and the instrument 200.

It is understandable that the specific embodiments described above are merely intended to explain the present application, but the protection scope of the present application is not limited thereto. Any variation, or replacement or combination made by those skilled in the art within the technical scope disclosed in the present application shall be covered within the protection scope of the present application.

**Claims**

1. An adjustment and positioning device for instrument, comprising a bracket fixedly connected with the instrument and a locking mechanism in locking engagement with the bracket, wherein

   the bracket comprises a rack disposed on a side face of the bracket;
   the locking mechanism comprises a sliding member configured to move toward or away from the rack in a normal direction of the side face of the bracket provided with the rack; and a plurality of locking plugs connected to the sliding member and extending in the normal direction; by taking an arrangement direction of a plurality of teeth in the rack as an extension direction of the rack, and an end of each of the teeth close to the sliding member as a tooth tip, in the extension direction of the rack, each tooth further includes a first tooth surface and a second tooth surface extending from the tooth tip away from the sliding member, and tilting toward opposite sides of the normal direction;

each of the plurality of locking plugs is connected to the sliding member via a first elastic element at a position further back relative to a tip end of the locking plug close to the rack in the extension direction of the locking plug, and the first elastic element allows a specified amount of deformation in the extension direction of the locking plug; under an elastic force of the first elastic element, the tip end of the locking plug close to the rack is capable of moving toward the rack and insert itself into a gap, i.e., tooth slot between two adjacent teeth of the rack;

a surface tangent to tooth tips of all teeth of the rack in the extension direction of the rack is regarded as a reference surface; and it is assumed that a corresponding spacing between centers of every two adjacent tooth tips on the reference surface in the extension direction of the rack, i.e., a tooth spacing, is $t_1$, and a corresponding spacing between tip ends of two adjacent locking plugs randomly selected from the plurality of locking plugs, close to the rack on the reference surface in the extension direction of the rack, i.e., a plug spacing, is $t_2$, wherein the plug spacing $t_2$ is a non-integer multiple of the tooth spacing $t_1$; and

accordingly, when the sliding member moves to a position closest to the rack, the first tooth surface of at least one tooth and the second tooth surface of at least one tooth in the rack respectively abut against at least one locking plug of the plurality of locking plugs.

2. The adjustment and positioning device for instrument according to claim 1, wherein

assuming the quantity of locking plugs in the plurality of locking plugs is denoted as x, $t_1$, $t_2$ and x satisfy the following Equation (1):

$$t_2 = \left(n \pm \frac{1}{x}\right) t_1 \quad \text{Equation (1)};$$

wherein
n is a natural number greater than or equal to 1.

3. The adjustment and positioning device for instrument according to claim 2, wherein
assuming in the extension direction of the rack, a is defined as a dimension of a narrowest part of the tip end closest to the rack, of a single locking plug of the plurality of locking plugs, and b is defined as a dimension of a widest part of the tooth slot, a and b satisfy Equation (2):

$$\frac{t_1}{x} \le b - a \quad \text{Equation (2)}.$$

4. The adjustment and positioning device for instrument according to claim 1, wherein
the sliding member is provided with a plurality of plug guide rails, and the plurality of plug guide rails and the plurality of locking plugs are of the same quantity, and are arranged in one-to-one correspondence; and accordingly, a single locking plug of the plurality of locking plugs is capable of moving toward or away from the rack via the corresponding plug guide rail, and the locking plug is limited by the plug guide rail on a section perpendicular to the extension direction of the locking plug.

5. The adjustment and positioning device for instrument according to any one of claims 1 to 4, wherein
the rack is in a curved shape, and when the bracket and the instrument are connected, the instrument is capable of being adjusted in position synchronously with the rack.

6. The adjustment and positioning device for instrument according to claim 5, wherein

the rack is in an arc shape, and with a radial direction of a circumference of the rack being a radial direction of the adjustment and positioning device for instrument, a direction perpendicular to a radial section of the rack being an axial direction, and a circumferential direction of the rack being a circumferential direction, when the bracket and the instrument are connected, the instrument is capable of rotating synchronously with the rack around an axis extending axially from a center of the circumference of the rack.

7. The adjustment and positioning device for instrument according to claim 6, wherein
the reference surface is an arc surface, and on the radial section of the rack, the tooth spacing $t_1$ is a corresponding circumferential arc length between the centers of every two adjacent tooth tips on the reference surface; and the plug spacing $t_2$ is a corresponding circumferential arc length between centers of the tip ends of two adjacent locking plugs randomly selected from the plurality of locking plugs, close to the rack, on the reference surface.

8. The adjustment and positioning device for instrument according to any one of claims 1 to 4, wherein
the tip end of a single locking plug of the plurality of locking plugs close to the rack is configured to be conical, trapezoidal, V-shaped, or spherical.

9. The adjustment and positioning device for instrument according to claim 8, wherein
the tip end of a single locking plug of the plurality of locking plugs close to the rack is configured to be conical, V-shaped, or trapezoidal, and an apex angle of a longitudinal section of the locking plug ranges from 20 to 150 degrees.

10. The adjustment and positioning device for instrument according to any one of claims 1 to 4, further comprising:
at least one roller/ball abutting against at least one side face of the bracket.

11. The adjustment and positioning device for instrument according to claim 10, wherein

at least a portion of the at least one roller/ball is located on the same side of the bracket as the rack, and
a portion of the bracket on the same side as the rack is provided with a blank region without the rack, and for abutting against the roller/ball on the same side.

12. The adjustment and positioning device for instrument according to claim 1, wherein
the sliding member abuts against the locking mechanism via a second elastic element at a position further back relative to a front end of the sliding member close to the rack in a movement direction of the sliding member, and the second elastic element allows a specified amount of deformation in the movement direction of the sliding member.

13. The adjustment and positioning device for instrument according to claim 12, wherein
when the sliding member moves to a locking position closest to the rack, an elastic force of the second elastic element is greater than or equal to a sum of elastic forces of the first elastic elements corresponding to each of the plurality of locking plugs abutting against the rack.

FIG. 1

FIG. 2

FIG. 3

FIG. 4

FIG. 5

FIG. 6

FIG. 7

FIG. 8

FIG. 9

FIG. 10

## INTERNATIONAL SEARCH REPORT

International application No.

**PCT/CN2024/076268**

| A. | CLASSIFICATION OF SUBJECT MATTER |
|---|---|

F16M11/00(2006.01)i

According to International Patent Classification (IPC) or to both national classification and IPC

| B. | FIELDS SEARCHED |
|---|---|

Minimum documentation searched (classification system followed by classification symbols)

IPC: F16M 11/-; A61B 17/-

Documentation searched other than minimum documentation to the extent that such documents are included in the fields searched

Electronic data base consulted during the international search (name of data base and, where practicable, search terms used)

CJFD; CNTXT; ENTXTC; WPABSC; VEN; DWPI; CNABS; WPABS: 智愈医疗, 调节, 定位, 支架, 医疗, 器械, 锁紧, 齿条, 滑动, 插头, 弹簧, 弹性元件, 整数, 倍, 精度, 自由度, adjust+, position+, bracket?, standing?, holder?, medical, lock+, tooth, rack?, slid+, plug?, pin?, spring?, elastic+, integer, precision, freedom

| C. | DOCUMENTS CONSIDERED TO BE RELEVANT |
|---|---|

| Category* | Citation of document, with indication, where appropriate, of the relevant passages | Relevant to claim No. |
|---|---|---|
| PX | CN 115823431 A (BEIJING ZHIYU MEDICAL TECHNOLOGY CO., LTD.) 21 March 2023 (2023-03-21)<br>claims 1-13 | 1-13 |
| A | CN 114052985 A (HANGZHOU DEJIN MEDICAL TECHNOLOGY CO., LTD.) 18 February 2022 (2022-02-18)<br>description, paragraphs 24-60, and figures 1-13 | 1-13 |
| A | CN 113413219 A (BEST MEDICAL TECHNOLOGY (BEIJING) CO., LTD.) 21 September 2021 (2021-09-21)<br>entire document | 1-13 |
| A | CN 115414215 A (SHANGHAI HUIHE MEDICAL INSTRUMENT CO., LTD.) 02 December 2022 (2022-12-02)<br>entire document | 1-13 |
| A | CN 205698057 U (LIU HONGXIAO et al.) 23 November 2016 (2016-11-23)<br>entire document | 1-13 |

☑ Further documents are listed in the continuation of Box C.　　☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **06 March 2024** | **23 March 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2024/076268** |

### C.    DOCUMENTS CONSIDERED TO BE RELEVANT

| Category* | Citation of document, with indication, where appropriate, of the relevant passages | Relevant to claim No. |
| --- | --- | --- |
| A | DE 102020133199 A1 (STORZ OLAF) 17 June 2021 (2021-06-17) <br> entire document | 1-13 |

Form PCT/ISA/210 (second sheet) (July 2022)

**INTERNATIONAL SEARCH REPORT**
Information on patent family members

International application No.

**PCT/CN2024/076268**

| Patent document cited in search report | | | Publication date (day/month/year) | Patent family member(s) | Publication date (day/month/year) |
|---|---|---|---|---|---|
| CN | 115823431 | A | 21 March 2023 | None | |
| CN | 114052985 | A | 18 February 2022 | None | |
| CN | 113413219 | A | 21 September 2021 | None | |
| CN | 115414215 | A | 02 December 2022 | None | |
| CN | 205698057 | U | 23 November 2016 | None | |
| DE | 102020133199 | A1 | 17 June 2021 | None | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202310110698